# EUROPÄISCHE PATENTSCHRIFT

(11) **EP 0 725 062 B1**
(45) Veröffentlichungstag und Bekanntmachung des Hinweises auf die Patenterteilung: **13.06.2001**
(21) Anmeldenummer: 96100764.8
(22) Anmeldetag: 19.01.1996
(51) Int. Cl.: C07D 213/70, C07C 323/65, C07D 239/38, C07D 239/10, C07D 277/16, C07D 249/12, A61K 31/155, A61K 31/44, A61K 31/425, A61K 31/505, A61K 31/41

(54) **4-Mercapto-benzoylguanidin-Derivate**
4-mercapto-benzoylguanidine derivatives
Dérivés de 4-mercapto-benzoylguanidine

(30) Priorität: 31.01.1995 DE 19502895
(43) Veröffentlichungstag der Anmeldung: 07.08.1996
(73) Patentinhaber: MERCK PATENT GmbH, 64293 Darmstadt (DE)
(72) Erfinder: Gericke, Rolf, Dr., D-64342 Seeheim (DE); Baumgarth, Manfred, Dr., D-64297 Darmstadt (DE); Beier, Norbert, Sr., D-64354 Reinheim (DE); Minck, Klaus-Otto, Dr., D-64372 Ober-Ramstadt (DE)

(56) Entgegenhaltungen:
- EP-A- 0 416 499
- EP-A- 0 589 336
- EP-A- 0 602 523
- EP-A- 0 640 588

## Beschreibung

Die Erfindung betrifft ortho-substituierte 4-Mercapto-benzoylguanidin-Derivate der Formel I 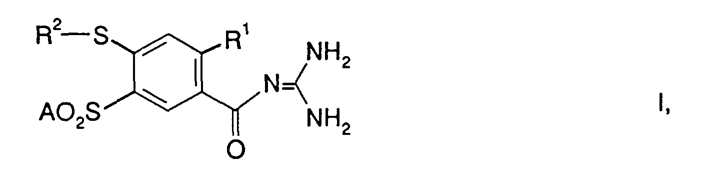 worin
- R¹: A, CF₃, CH₂F, CHF₂, oder C₂F₅,
- R²: H, A, Cycloalkyl mit 3 bis 7 C-Atomen, Ph oder Het,
- Het: einen ein- oder zweikernigen gesättigten, ungesättigten oder aromatischen Heterocyclus mit 1 bis 4 N-, O-, und/oder S-Atomen, über N oder C gebunden, der unsubstituiert oder ein-, zwei- oder dreifach durch Hal, CF₃, A, OH, OA, SH, SA, NH₂, NHA, NA₂, CN, NO₂ und/oder Carbonylsauerstoff substituiert sein kann,
- A: Alkyl mit 1 bis 6 C-Atomen,
- Hal: F, Cl, Br oder I und
- Ph: unsubstituiertes oder ein-, zwei- oder dreifach durch A, OA, NH₂, NHA, NA₂, F, Cl, Br und/oder CF₃ substituiertes Phenyl
bedeuten,
sowie deren physiologisch unbedenkliche Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen.

Bei den neuen Verbindungen handelt es sich um Inhibitoren des zellulären Na⁺/H⁺-Antiporters, d.h. um Wirkstoffe, die den Na⁺/H⁺-Austauschmechanismus der Zellen hemmen (Düsing et al., Med. Klin. 87, 378-384 (1992)) und die somit gute Antiarrhythmika darstellen, die sich insbesondere zur Behandlung von Arrhythmien eignen, die als Folge von Sauerstoffmangel auftreten.

Der bekannteste Wirkstoff der Gruppe der Acylguanidine ist Amilorid. Diese Substanz zeigt jedoch in erster Linie eine blutdrucksenkende und saluretische Wirkung, was insbesondere bei der Behandlung von Herz-Rhythmus-Störungen unerwünscht ist, während die antiarrhythmischen Eigenschaften nur sehr schwach ausgeprägt sind.

Darüber hinaus kennt man strukturell ähnliche Verbindungen beispielsweise aus EP 04 16 499.

Benzoylguanidine mit anderen Substituenten R¹ sind in EP 602523 und EP 589336 beschrieben.

Gegenstand der Erfindung sind Verbindungen der Formel I sowie ihre physiologisch unbedenklichen Salze.

Die erfindungsgemäßen Substanzen der vorliegenden Anmeldung zeigen eine gute kardioprotektive Wirkung und eignen sich daher besonders zur Infarktbehandlung, Infarktprophylaxe und zur Behandlung von Angina pectoris. Ferner wirken die Substanzen allen pathologischen hypoxischen und ischämischen Schädigungen entgegen, so daß die dadurch primär oder sekundär verursachten Krankheiten behandelt werden können. Die Wirkstoffe sind ebenfalls für präventive Anwendungen gut geeignet.

Aufgrund der protektiven Wirkungen dieser Substanzen bei pathologischen, hypoxischen oder ischämischen Situationen resultieren daraus weitere Anwendungsmöglichkeiten bei chirurgischen Eingriffen zum Schutz zeitweilig minderversorgter Organe, bei Organtransplantationen zum Schutz der entnommenen Organe, bei angioplastischen Gefäß- oder Herzeingriffen, bei Ischämien des Nervensystems, bei der Therapie von Schockzuständen und zur präventiven Verhinderung der essentiellen Hypertonie.

Ferner können die Verbindungen auch als Therapeutika bei durch Zellproliferation bedingten Erkrankungen wie Arteriosklerose, diabetische Spätkomplikationen, Tumorerkrankungen, fibrotischen Erkrankungen, insbesondere von Lunge, Leber und Nieren sowie Organhypertrophien und -hyperplasien, eingesetzt werden. Darüber hinaus eignen sich die Substanzen zur diagnostischen Anwendung zur Erkennung von Krankheiten, die von einer gesteigerten Aktivität des Na+/H+-Antiporters z.B. in Erythrozyten, Thrombozyten oder Leukozyten begleitet werden.

Die Wirkungen der Verbindungen können mit Hilfe an sich bekannter Methoden ermittelt werden, wie sie z.B. von N. Escobales and J. Figueroa in J. Membrane Biol. 120, 41-49 (1991) oder von L. Counillon, W. Scholz, H.J. Lang und J. Pouysségur in Mol. Pharmacol. 44, 1041-1045 (1993) angegeben werden.

Als Versuchstiere eignen sich z.B. Mäuse, Ratten, Meerschweinchen, Hunde, Katzen, Affen oder Schweine.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe Verwendung finden.

In den angegebenen Formeln bedeutet A eine verzweigte oder unverzweigte Alkylgruppe mit 1-6, bevorzugt 1-4, insbesondere 1, 2 oder 3 C-Atomen, im einzelnen vorzugsweise Methyl, ferner bevorzugt Ethyl, Propyl, Isopropyl, Butyl, Isobutyl, weiterhin bevorzugt sek.-Butyl, tert.-Butyl, Pentyl, Isopentyl (3-Methylbutyl), Hexyl oder Isohexyl (4-Methylpentyl).

R¹ bedeutet vorzugsweise A, insbesondere Methyl oder Ethyl.

R² A, Phenyl, 2-, 3- oder 4-Chlorphenyl, 2-, 3- oder 4-Fluorphenyl oder Het. Besonders bevorzugt steht Het neben den unten angegebenen Bedeutungen für Pyridyl, Pyrimidyl, Triazolyl, Thiazolyl oder die teilweise oder vollständig hydrierten Abkömmlinge dieser Reste, die auch wie angegeben substituiert sein können.

Ph bedeutet vorzugsweise unsubstituiertes oder einfach durch F, Cl, Br, A, OA, NH₂, NHA, NA₂ oder CF₃ substituiertes Phenyl.

Hal bedeutet vorzugsweise F, Cl oder Br.

Het ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4-oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3-oder -5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3-oder 4-4H-Thiopyranyl, 3- oder 4-Pyridazinyl, Pyrazinyl, 2-, 3-, 4-, 5-, 6-oder 7-Benzofuryl, 2-, 3-, 4-, 5-, 6- oder 7-Benzothienyl, 1-, 2-, 3-, 4-, 5-, 6- oder 7-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolinyl, 1-, 3-, 4-, 5-, 6-, 7- oder 8-Isochinolinyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolinyl, 2-, 4-, 5-, 6- 7- oder 8-Chinazolinyl. Die heterocyclischen Reste können auch teilweise oder vollständig hydriert sein.

Het kann also z.B. auch bedeuten 2,3-Dihydro-2-, -3-, -4- oder -5-furyl, 2,5-Dihydro-2-, -3-, -4- oder -5-furyl, Tetrahydro-2- oder -3-furyl, 1,3-Dioxolan-4-yl, Tetrahydro-2- oder -3-thienyl, 2,3-Dihydro-1-, -2-, -3-, -4-oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3-oder -4-pyridyl, 1,2,3,4-Tetrahydro-1-, -2-, -3-, -4-, -5- oder -6-pyridyl, 1,2,3,6-Tetrahydro-1-, -2-, -3-, -4-, -5- oder -6-pyridyl, 1-, 2-, 3-oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3-oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolinyl, 1,2,3,4-Tetrahydro-1-, -2-, -3-, -4-, -5-, -6-, -7- oder-8-isochinolinyl.

Allgemein gilt, daß sämtliche Reste wie z.B. A, die mehrfach im Molekül auftreten können, gleich oder verschieden, d.h. unabhängig voneinander sein können.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln la bis Ih ausgedrückt werden, die der Formel I entsprechend und worin die nicht näher bezeichneten Reste die bei der Formel I angegebene Bedeutung haben, worin jedoch
- in la: R¹ Methyl oder Ethyl bedeutet;
- in Ib: R¹ Methyl oder Ethyl und R² A bedeutet;
- in Ic: R¹ Methyl oder Ethyl und R² 2-Thiazolyl, 4,5-Dihydro-thiazol-2-yl, 1,2,4-Triazol-3-yl oder 1,2,4-(4-Methyltriazol-3-yl) bedeutet;
- in Id: R¹ Methyl oder Ethyl, und R² Imidazolyl, Pyridyl, oder Pyrimidinyl bedeutet;
- in le: R¹ Methyl oder Ethyl und R² Pyridyl oder Pyrimidinyl bedeutet;
- in If: R¹ Methyl oder Ethyl und R² Phenyl, Fluorphenyl oder Chlorphenyl bedeutet;
- in Ig: R¹ Methyl oder Ethyl und R² Methyl, Ethyl, Propyl, Isopropyl oder Butyl bedeutet;
- in Ih: R¹ Methyl oder Ethyl und R² Cylcohexyl oder Cyclopentyl bedeutet.

Ferner sind jene Verbindungen besonders bevorzugt, die die unter la bis Ih genannten bevorzugten Bedeutungen haben, bei denen aber zusätzlich -SO₂A für Methylsulfonyl steht.

Gegenstand der Erfindung ist ferner ein Verfahren zur Herstellung der Verbindungen der Formel I gemäß Anspruch 1, sowie von deren Salzen, dadurch gekennzeichnet, daß man eine Verbindung der Formel II 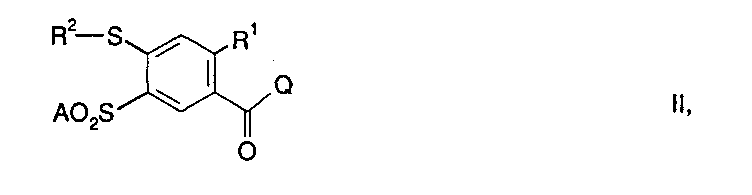 worin R¹, R² und A die zuvor angegebenen Bedeutungen haben
und
- Q: Cl, Br, OA, O-CO-A, O-CO-Ph, OH oder eine andere reaktionsfähige veresterte OH-Gruppe bzw. leicht nucleophil substituierbare Abgangsgruppe bedeutet,
mit Guanidin umsetzt,
oder daß man ein Benzoylguanidin der Formel III 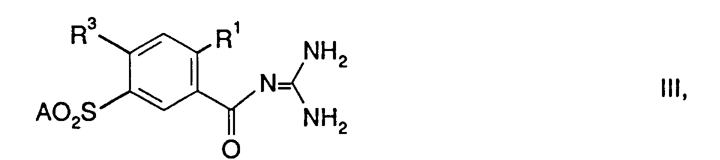 worin R¹ und A die zuvor angegebenen Bedeutungen besitzen,
und
- R³: F, Cl, Br, I oder eine andere geeignete Abgangsgruppe
bedeutet,
mit einer Verbindung der Formel IV

R²-S-H IV

worin
- R²: die angegebenen Bedeutungen besitzt,
oder einer daraus ableitbaren salzartigen Verbindung, einem Thiolat,
umsetzt,
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C-und/oder C-N-Bindung(en) enthält, mit einem reduzierenden Mittel behandelt,
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt
und/oder daß man eine erhaltene Base der Formel I durch Behandeln mit einer Säure in eines ihrer Salze umwandelt.

Die Verbindungen der Formel I werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; Organic Reactions, John Wiley & Sons, Inc., New York; sowie in der oben angegebenen Patentanmeldung) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise werden Verbindungen der Formel I hergestellt, indem man ein aktiviertes Carbonsäurederivat der Formel II, wobei Q besonders bevorzugt Cl oder -O-CH₃ ist, mit Guanidin umsetzt. Besonders geeignet sind auch Reaktionsvarianten, bei denen die freie Carbonsäure II (Q = OH) in an sich bekannter Weise zu dem jeweiligen aktivierten Derivat umgesetzt und dieses dann direkt, ohne Zwischenisolierung, mit Guanidin zur Reaktion gebracht wird. Methoden, bei denen eine Zwischenisolierung entbehrlich ist, sind beispielsweise eine Aktivierung mit Carbonyldiimidazol, Dicyclohexylcarbodiimid oder die Mukayama-Variante (Angew. Chem. 91, 788-812 (1979)).

Die Carbonsäuren der Formel II werden z.B. durch nucleophile aromatische Substitution ausgehend von geeigneten Benzoesäurederivaten mit entsprechenden Thiolen bzw. Thiophenolen hergestellt. Die Umsetzung erfolgt in Analogie zu der Reaktion der Verbindungen III und IV. Sie wird nachstehend beschrieben.

Die Umsetzung eines reaktionsfähigen Carbonsäurederivates der Formel II mit Guanidin erfolgt in an sich bekannter Weise vorzugsweise in einem protischen oder aprotischen polaren oder unpolaren inerten organischen Lösungsmittel.

Geeignete Lösungsmittel werden nachfolgend für die Umsetzung der Verbindungen III und IV genannt. Besonders bevorzugte Solventien sind jedoch Methanol, THF, Dimethoxyethan, Dioxan, Wasser oder daraus herstellbare Gemische. Als Reaktionstemperatur sind beispielsweise Temperaturen zwischen 20° und dem Siedepunkt des Lösungsmittels geeignet. Die Reaktionszeiten liegen zwischen 5 Min. und 12 Std.. Es ist zweckmäßig, bei der Reaktion einen Säurefänger einzusetzen. Hierzu eignen sich jegliche Arten von Basen, die die Reaktion selbst nicht stören. Besonders geeignet ist jedoch die Verwendung von anorganischen Basen wie Kaliumcarbonat oder von organischen Basen wie Triethylamin oder Pyridin oder aber ein Überschuß des Guanidins.

Verbindungen der Formel I gemäß Anspruch 1 können ferner hergestellt werden, indem man ein Benzoylguanidin der Formel III mit einer Verbindung der Formel IV umsetzt. Die Ausgangsstoffe der Formel III können auf einfache Weise durch Umsetzung von entsprechend substituierten Benzoesäuren oder daraus ableitbaren reaktionsfähigen Säurederivaten, wie z.B. Säurehalogeniden, Estern oder Anhydriden, mit Guanidin, unter Reaktionsbedingungen wie sie für die Amidherstellung an sich bekannt und allgemein üblich sind, hergestellt werden. Besonders geeignet sind wiederum solche Reaktionsvarianten, wie sie zuvor für die Umsetzung von Verbindung II mit Guanidin angegeben werden.

Die Thiole oder Thiophenole bzw. Thiolate der Formel IV sind ebenso wie die Methoden zu ihrer Herstellung an sich bekannt. Sofern sie nicht bekannt sind, können sie nach den an sich bekannten Methoden hergestellt werden.

Die Herstellung der Verbindung II sowie die Umsetzung der Verbindung III mit einer Verbindung der Formel IV erfolgt in an sich bekannter Weise, bevorzugt in einem protischen oder aprotischen polaren inerten organischen Lösungsmittel.

Eine bevorzugte Variante besteht allerdings auch darin, daß man die Reaktionspartner direkt, ohne Zusatz eines Lösungsmittels, miteinander zur Reaktion bringt.

Bei der Herstellung von II oder bei der Umsetzung von III mit IV ist es ebenfalls zweckmäßig, in Gegenwart einer Base oder mit einem Überschuß der basischen Komponente zu arbeiten. Als Basen eignen sich bevorzugt z.B. Alkalimetall- oder Erdalkalimetallhydroxide, -carbonate, -alkoholate oder organische Basen wie Triethylamin oder Pyridin, die auch im Überschuß angewendet werden und dann gleichzeitig als Lösungsmittel dienen können.

Als inerte Lösungsmittel eignen sich insbesondere Alkohole wie Methanol, Ethanol, Isopropanol, n-Butanol oder tert.-Butanol; Ether wie Diethylether, Diisopropylether, THF oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat; Amide wie Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander.

Weiterhin können die Verbindungen der Formel I erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer OH-Gruppe eine OR"-Gruppe tragen, worin R" eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. 2,4-Dinitrophenyl (DNP)), Aralkoxymethyl- (z.B. Benzyloxymethyl (BOM)) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden

Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluoyl; Aryloxyalkanoyl wie Phenoxyacetyl; Alkoxycarbonyl wie Methoxycarbonyl, Ethoxycarbonyl, 2,2,2-Trichlorethoxycarbonyl, Isopropoxycarbonyl, tert.-Butoxycarbonyl (BOC), 2-Jodethoxycarbonyl; Aralkyloxycarbonyl wie Benzyloxycarbonyl (CBZ), 4-Methoxybenzyloxycarbonyl, 9-Fluorenylmethoxycarbonyl (FMOC). Bevorzugte Aminoschutzgruppen sind BOC, DNP und BOM, ferner CBZ, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden hergestellt werden, wie sie z.B. in den genannten Standardwerken und Patentanmeldungen beschrieben sind, z.B. durch Umsetzung von Verbindungen, die den Formeln II und III entsprechen, wobei jedoch mindestens eine dieser Verbindungen eine Schutzgruppe anstelle eines H-Atoms enthält.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich.

Als inerte Lösungsmittel eignen sich vorzugsweise organische, beispielsweise Carbonsäuren wie Essigsäure, Ether wie Tetrahydrofuran (THF) oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°; vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCI in Dioxan bei 15-60° abgespalten weden, die FMOC-Gruppe mit einer etwa 5-20%igen Lösung von Dimethylamin, Diethylamin oder Piperidin in DMF bei 15-50°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B.

Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Eine Base der Formel I kann ferner mit einer Säure in das zugehörige Säureadditionssalz übergeführt werden. Für diese Umsetzung kommen Säuren in Frage, die physiologisch unbedenkliche Salze liefern. So können anorganische Säuren verwendet werden, z.B. Schwefelsäure. Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalinmono- und -disulfonsäuren, Laurylschwefelsäure.

Die Verbindungen der Formel I und ihre physiologisch unbedenklichen Salze können zur Herstellung pharmazeutischer Zubereitungen verwendet werden, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlenhydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z.B. Lösungen in Alkoholen wie Ethanol oder Isopropanol, Acetonitril, DMF, Dimethylacetamid, 1,2-Propandiol oder deren Gemischen untereinander und/oder mit Wasser) oder Puder. Die neuen Verbindungen können auch lyophilisiert und die erhaltenen Lyophilisate z.B. zur Herstellung von Injektionspiräparaten verwendet werden.

Insbesondere für die topische Anwendung kommen auch liposomale Zubereitungen in Betracht. Die angegebenen Zubereitungen können sterilisiert sein und/oder Hilfsstoffe wie Geleit-, Konservierungs-, Stabilisierungs- und/oder Netzmittel, Emulgatoren, Salze zur Beeinflussung des osmotisches Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems. Sie eignen sich daher zur Behandlung von Arrhythmien, insbesondere wenn diese durch Sauerstoffmangel hervorgerufen werden, von Angina pectoris, Infarkten, Ischämien des Nervensystems wie z.B. Schlaganfall oder Hirnödeme, von Schockzuständen und zur Präventivbehandlung.

Die Substanzen können ferner als Therapeutika bei Erkrankungen eingesetzt werden, bei denen Zellproliferationen eine Rolle spielen wie Arteriosklerose, diabetische Spätkomplikationen, Tumorerkrankunken, Fibrosen sowie Organhypertrophien und -hyperplasien, insbesondere bei Erkrankungen der Prostata.

Dabei werden die erfindungsgemäßen Substanzen in der Regel in Analogie zu bekannten Antiarrhythmika, z.B. Aprindin, verabreicht, vorzugsweise in Dosierungen zwischen etwa 0,01 und 5 mg, insbesondere zwischen 0,02 und 0,5 mg pro Dosierungseinheit. Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachfolgenden Beispielen bedeutet "übliche Aufarbeitung":

Man gibt, falls erforderlich, Wasser hinzu, extrahiert mit einem organischen Lösungsmittel wie Ethylacetat, trennt ab, trocknet die organische Phase über Natriumsulfat, filtriert, dampft ein und reinigt durch Chromatographie und/oder Kristallisation.

### Beispiel 1

Zu einer Lösung von 1,5 g Guanidin, gelöst in 20 ml Ethylenglykoldimethylether, gibt man bei Raumtemperatur tropfenweise 1,8 g 2-Methyl-4-(4-pyridylthio)-5-methylsulfonyl-benzoesäurechlorid [erhältlich durch Umsetzung von 2-Methyl-4-chlor-5-methylsulfonyl-benzoesäure mit 4-Mercapto-pyridin in Gegenwart von NaOCH₃ bei 180° und anschließende Chlorierung mit SOCl₂], in 20 ml Ethylenglykoldimethylether gelöst, hinzu und rührt drei Stunden bei 25°. Anschließend arbeitet man wie üblich auf und reinigt durch Chromatographie an Kieselgel (Essigsäureethylester + 15 % Methanol). Man erhält das N-Diaminomethylen-2-methyl-4-(4-pyridylthio)-5-methylsulfonyl-benzamid in Form eines zähflüssigen Öls, M⁺+1(FAB) = 365.

Analog erhält man durch Umsetzung von Guanidin
mit 2-Methyl-4-(4-chlorphenylthio)-5-methylsulfonyl-benzoesäurechlorid das
   N-Diaminomethylen-2-methyl-4-(4-chlorphenylthio)-5-methylsulfonylbenzamid, F. 245-247° (F. > 250° Methansulfonat);
mit 2-Methyl-4-(3-chlorphenylthio)-5-methylsulfonyl-benzoesäurechlorid das
   N-Diaminomethylen-2-methyl-4-(3-chlorphenylthio)-5-methylsulfonylbenzamid, F. 198-202°; F. 213-215° (Methansulfonat);
mit 2-Methyl-4-(2-chlorphenylthio)-5-methylsulfonyl-benzoesäurechlorid das
   N-Diaminomethylen-2-methyl-4-(2-chlorphenylthio)-5-methylsulfonylbenzamid, F. 184-187° (F. > 250° Methansulfonat);
mit 2-Methyl-4-phenylthio-5-methylsulfonyl-benzoesäurechlorid das
   N-Diaminomethylen-2-methyl-4-phenylthio-5-methylsulfonylbenzamid, F. 125-130°;
mit 2-Methyl-4-(4-fluorphenylthio)-5-methylsulfonyl-benzoesäurechlorid das
   N-Diaminomethylen-2-methyl-4-(4-fluorphenylthio)-5-methylsulfonylbenzamid;
mit 2-Methyl-4-(3-fluorphenylthio)-5-methylsulfonyl-benzoesäurechlorid das
   N-Diaminomethylen-2-methyl-4-(3-fluorphenylthio)-5-methylsulfonylbenzamid;
mit 2-Methyl-4-(2-fluorphenylthio)-5-methylsulfonyl-benzoesäurechlorid das
   N-Diaminomethylen-2-methyl-4-(2-fluorphenylthio)-5-methylsulfonylbenzamid;
mit 2-Methyl-4-(3-pyridylthio)-5-methylsulfonyl-benzoesäurechlorid das
   N-Diaminomethylen-2-methyl-4-(3-pyridylthio)-5-methylsulfonylbenzamid;
mit 2-Methyl-4-(2-pyrimidinylthio)-5-methylsulfonyl-benzoesäurechlorid das
   N-Diaminomethylen-2-methyl-4-(2-pyrimidinylthio)-5-methylsulfonylbenzamid;
mit 2-Methyl-4-(2-pyridyllhio)-5-methylsulfonyl-benzoesäurechlorid das
   N-Diaminomethylen-2-methyl-4-(2-pyridylthio)-5-methylsulfonylbenzamid;
mit 2-Methyl-4-[2-(1,4,5,6-tetrahydropyrimidinylthio)]-5-methylsulfonylbenzoesäurechlorid das
   N-Diaminomethylen-2-methyl-4-[2-(1,4,5,6-tetrahydropyrimidinylthio)]-5-methylsulfonyl-benzamid;
mit 2-Methyl-4-(4,5-dihydro-thiazol-2-yl-thio)-5-methylsulfonylbenzoesäurechlorid das
   N-Diaminomethylen-2-methyl-4-(4,5-dihydro-thiazol-2-yl-thio)]-5-methylsulfonyl-benzamid;
mit 2-Methyl-4-[2-(4-N-methyl-1,2,4-triazol-3-yl-thio)]-5-methylsulfonylbenzoesäurechlorid das
   N-Diaminomethylen-2-methyl-4-[2-(4-N-methyl-1,2,4-triazol-3-yl-thio)]-5-methylsulfonyl-benzamid.

### Beispiel 2

0,9 g N-Diaminomethylen-2-methyl-4-(4-pyridylthio)-5-methylsulfonylbenzamid [erhältlich nach Bsp. 1] werden in 100 ml H₂O suspendiert, mit 1 molarer wäßriger HCI-Lösung aufgelöst, und anschließend gefriergetrocknet. Man erhält das N-Diaminomethylen-2-methyl-4-(4-pyridylthio)-5-methylsulfonyl-benzamid, Dihydrochlorid, F. > 250°.

Analog erhält man durch Behandlung mit wäßriger HCI und anschließende Gefriertrocknung
aus N-Diaminomethylen-2-methyl-4-phenylthio-5-methylsulfonyl-benzamid das
   N-Diaminomethylen-2-methyl-4-phenylthio-5-methylsulfonyl-benzamid, Hydrochlorid, F. > 260°;
aus N-Diaminomethylen-2-methyl-4-(2-pyridylthio)-5-methylsulfonylbenzamid das
   N-Diaminomethylen-2-methyl-4-(2-pyridylthio)-5-methylsulfonylbenzamid, Dihydrochlorid;
aus N-Diaminomethylen-2-methyl-4-(3-fluorphenylthio)-5-methylsulfonylbenzamid das
   N-Diaminomethylen-2-methyl-4-(3-fluorphenylthio)-5-methylsulfonylbenzamid, Hydrochlorid;
aus N-Diaminomethylen-2-methyl-4-(4-fluorphenylthio)-5-methylsulfonylbenzamid das
   N-Diaminomethylen-2-methyl-4-(4-fluorphenylthio)-5-methylsulfonylbenzamid, Hydrochlorid;
aus N-Diaminomethylen-2-methyl-4-(4-chlorphenylthio)-5-methylsulfonylbenzamid das
   N-Diaminomethylen-2-methyl-4-(4-chlorphenylthio)-5-methylsulfonylbenzamid, Hydrochlorid;
aus N-Diaminomethylen-2-methyl-4-methylthio-5-methylsulfonyl-benzamid das
   N-Diaminomethylen-2-methyl-4-methylthio-5-methylsulfonylbenzamid; Hydrochlorid, F. > 260°;
aus N-Diaminomethylen-2-methyl-4-(2-chlorphenylthio)-5-methylsulfonylbenzamid das
   N-Diaminomethylen-2-methyl-4-(2-chlorphenylthio)-5-methylsulfonylbenzamid, Hydrochlorid.

### Beispiel 3

2,9 g N-Diaminomethylen-2-methyl-4-chlor-5-methylsulfonyl-benzamid [erhältlich durch Umsetzung von 2-Methyl-4-chlor-5-methylsulfonylbenzoesäurechlorid mit Guanidin nach Bsp. 1] und 700 mg Natriumthiomethanolat in 30 ml DMF werden zwei Stunden bei 90° gerührt. Anschließend werden 30 ml Eiswasser zugegeben und die Reaktionsmischung mit 20 ml 1 n HCI angesäuert. Der entstan-dene Niederschlag wird abgesaugt und das Rohprodukt über Kieselgel chromatographisch gereinigt (Essigsäureethylester + 10% Methanol). Man erhält das N-Diaminomethylen-2-methyl-4-methylthio-5-methylsulfonyl-benzamid, F. 220-222°.

Analog erhält man durch Umsetzung von N-Diaminomethylen-2-methyl-4-chlor-5-methylsulfonyl-benzam id
mit Na-thiopropanolat das
   N-Diaminomethylen-2-methyl-4-propylthio-5-methylsulfonylbenzamid, F. 215-218°; F. 195-197° (Methansulfonat);
mit Na-thioisopropanolat das
   N-Diaminomethylen-2-methyl-4-isopropylthio-5-methylsulfonylbenzamid, F. 185-186° (Methansulfonat);
mit Na-thioethanolat das
   N-Diaminomethylen-2-methyl-4-ethylthio-5-methylsulfonyl-benzamid, F. 238-240°; F. 152-154° (Metansulfonat);
mit Na-thio-tert.-butanolat das
   N-Diaminomethylen-2-methyl-4-tert.-butylthio-5-methylsulfonylbenzamid, F. 110-115°, F. 200-202° (Methansulfonat);
mit Na-Cyclohexylthiolat das
   N-Diaminomethylen-2-methyl-4-cyclohexylthio-5-methylsulfonylbenzamid;
mit Na-Cyclopentylthiolat das
   N-Diaminomethylen-2-methyl-4-cyclopentylthio-5-methylsulfonylbenzamid;

Analog erhält man durch Umsetzung von N-Diaminomethylen-2-ethyl-4-chlor-5-methylsulfonyl-benzamid
mit Na-thiomethanolat das
   N-Diaminomethylen-2-ethyl-4-methylthio-5-methylsulfonyl-benzamid;
mit Na-thiopropanolat das
   N-Diaminomethylen-2-ethyl-4-propylthio-5-methylsulfonyl-benzamid;
mit Na-thioisopropanolat das
   N-Diaminomethylen-2-ethyl-4-isopropylthio-5-methylsulfonylbenzamid;
mit Na-thioethanolat das
   N-Diaminomethylen-2-ethyl-4-ethylthio-5-methylsulfonyl-benzamid;
mit Na-Cyclohexylthiolat das
   N-Diaminomethylen-2-ethyl-4-cyclohexylthio-5-methylsulfonylbenzamid;
mit Na-Cyclopentylthiolat das
   N-Diaminomethylen-2-ethyl-4-cyclopentylthio-5-methylsulfonylbenzamid.

### Beispiel 4

Analog Beispiel 1 erhält man durch Umsetzung von Guanidin mit 2-Ethyl-4-(4-pyridylthio)-5-methylsulfonyl-benzoesäurechlorid [erhältlich durch Umsetzung von 2-Ethyl-4-chlor-5-methylsulfo-nyl-benzoesäure mit 4-Mercapto-pyridin in Gegenwart von NaOCH₃ bei 180° und anschließende Chlorierung mit SOCl₂] das N-Diaminomethylen-2-ethyl-4-(4-pyridylthio)-5-methylsulfonyl-benzamid.

Analog erhält man durch Umsetzung von Guanidin
mit 2-Ethyl-4-(4-chlorphenylthio)-5-methylsulfonyl-benzoesäurechlorid das
   N-Diaminomethylen-2-ethyl-4-(4-chlorphenylthio)-5-methylsulfonylbenzamid;
mit 2-Ethyl-4-(3-chlorphenylthio)-5-methylsulfonyl-benzoesäurechlorid das
   N-Diaminomethylen-2-ethyl-4-(3-chlorphenylthio)-5-methylsulfonylbenzamid;
mit 2-Ethyl-4-(2-chlorphenylthio)-5-methylsulfonyl-benzoesäurechlorid das
   N-Diaminomethylen-2-ethyl-4-(2-chlorphenylthio)-5-methylsulfonylbenzamid;
mit 2-Ethyl-4-phenylthio-5-methylsulfonyl-benzoesäurechlorid das
   N-Diaminomethylen-2-ethyl-4-phenylthio-5-methylsulfonyl-benzamid;
mit 2-Ethyl-4-(4-fluorphenylthio)-5-methylsulfonyl-benzoesäurechlorid das
   N-Diaminomethylen-2-ethyl-4-(4-fluorphenylthio)-5-methylsulfonylbenzamid;
mit 2-Ethyl-4-(3-fluorphenylthio)-5-methylsulfonyl-benzoesäurechlorid das
   N-Diaminomethylen-2-ethyl-4-(3-fluorphenylthio)-5-methylsulfonylbenzamid;
mit 2-Ethyl-4-(2-fluorphenylthio)-5-methylsulfonyl-benzoesäurechlorid das
   N-Diaminomethylen-2-ethyl-4-(2-fluorphenylthio)-5-methylsulfonylbenzamid;
mit 2-Ethyl-4-(3-pyridylthio)-5-methylsulfonyl-benzoesäurechlorid das
   N-Diaminomethylen-2-ethyl-4-(3-pyridylthio)-5-methylsulfonylbenzamid;
mit 2-Ethyl-4-(2-pyrimidinylthio)-5-methylsulfonyl-benzoesäurechlorid das
   N-Diaminomethylen-2-ethyl-4-(2-pyrimidinylthio)-5-methylsulfonylbenzamid;
mit 2-Ethyl-4-(2-pyridylthio)-5-methylsulfonyl-benzoesäurechlorid das
   N-Diaminomethylen-2-ethyl-4-(2-pyridylthio)-5-methylsulfonylbenzamid;
mit 2-Ethyl-4-[2-(1,4,5,6-tetrahydropyrimidinylthio)]-5-methylsulfonylbenzoesäurechlorid das
   N-Diaminomethylen-2-ethyl-4-[2-(1,4,5,6-tetrahydropyrimidinylthio)]-5-methylsulfonyl- benzamid;
mit 2-Ethyl-4-(4,5-dihydro-thiazol-2-yl-thio)-5-methylsulfonyl-benzoesäurechlorid das
   N-Diaminomethylen-2-ethyl-4-(4,5-dihydro-thiazol-2-yl-thio)]-5-methylsulfonyl-benzamid;
mit 2-Ethyl-4-[2-(4-N-methyl-1,2,4-triazol-3-yl-thio)]-5-methylsulfonylbenzoesäurechlorid das
   N-Diaminomethylen-2-ethyl-4-[2-(4-N-methyl-1,2,4-triazol-3-yl-thio)]-5-methylsulfonyl-benzamid.

Die nachfolgenden Beispiele betreffen pharmazeutische Zubereitungen:

### Beispiel A: Injektionsgläser

Eine Lösung von 100 g eines Wirkstoffes der Formel I und 5 g Dinatrium-hydrogenphosphat werden in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. 4-Mercapto-benzoylguanidine der Formel I 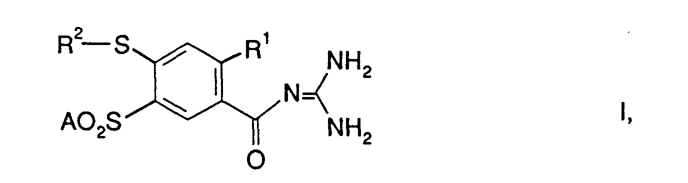 worin
R¹ A, CF₃, CH₂F, CHF₂ oder C₂F₅,
R² H, A, Cycloalkyl mit 3 bis 7 C-Atomen, Ph oder Het,
Het einen ein- oder zweikernigen gesättigten, ungesättigten oder aromatischen Heterocyclus mit 1 bis 4 N-, O-, und/oder S-Atomen, über N oder C gebunden, der unsubstituiert oder ein-, zwei- oder dreifach durch Hal, CF₃, A, OH, OA, SH, SA, NH₂, NHA, NA₂, CN, NO₂ und/oder Carbonylsauerstoff substituiert sein kann,
A Alkyl mit 1 bis 6 C-Atomen,
Hal F, Cl, Br oder I und
Ph unsubstituiertes oder ein-, zwei- oder dreifach durch A, OA, NH₂, NHA, NA₂, F, Cl, Br und/oder CF₃ substituiertes Phenyl
bedeuten,
sowie deren physiologisch unbedenkliche Salze.

2. (a) Diaminomethylen-2-methyl-4-(4-pyridylthio)-5-methylsulfonylbenzamid;
(b) N-Diaminomethylen-2-methyl-4-methylthio-5-methylsulfonylbenzamid;
(c) N-Diaminomethylen-2-methyl-4-isopropylthio-5-methylsulfonylbenzamid;
(d) N-Diaminomethylen-2-methyl-4-phenylthio-5-methylsulfonylbenzamid;
(e) N-Diaminomethylen-2-methyl-4-cyclohexylthio-5-methylsulfonylbenzamid;
(f) N-Diaminomethylen-2-methyl-4-(3-chlorphenylthio)-5-methylsulfonyl-benzamid;
(g) N-Diaminomethylen-2-ethyl-4-(4,5-dihydro-thiazol-2-yl-thio)-5-methylsulfonyl-benzamid
gemäß Anspruch 1, sowie deren physiologisch unbedenkliche Salze.

3. Verfahren zur Herstellung von Alkylbenzoylguanidin-Derivaten der Formel I gemäß Anspruch 1 sowie von deren Salzen, dadurch gekennzeichnet, daß man eine Verbindung der Formel II 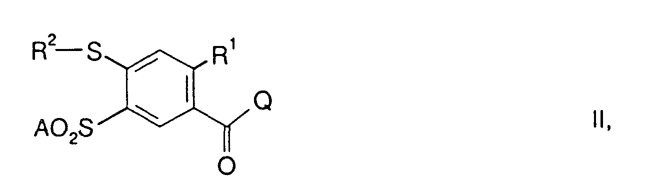 worin R¹, R² und A die zuvor angegebenen Bedeutungen haben
und
Q Cl, Br, OA, O-CO-A, O-CO-Ph, OH oder eine andere reaktionsfähige veresterte OH-Gruppe bzw. leicht nucleophil substituierbare Abgangsgruppe bedeutet,
mit Guanidin umsetzt,
oder daß man ein Benzoylguanidin der Formel III 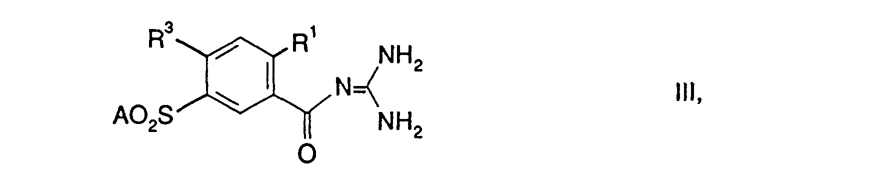 worin R¹ und A die zuvor angegebenen Bedeutungen besitzen,
und
R³ F, Cl, Br, I oder eine andere geeignete Abgangsgruppe bedeutet,
mit einer Verbindung der Formel IV
R²-S-H IV,
worin R² die angegebene Bedeutung besitzt,
oder einer daraus ableitbaren salzartigen Verbindung, einem Thiolat, umsetzt,
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt,
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt
und/oder daß man eine erhaltene Base der Formel I durch Behandeln mit einer Säure in eines ihrer Salze umwandelt.

4. Verfahren zur Herstellung pharmazeutischer Zubereitungen, dadurch gekennzeichnet, daß man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der allgemeinen Formel I gemäß Anspruch 1 und/oder einem ihrer physiologisch unbedenklichen Salze.

6. Verwendung von Verbindungen der Formel I nach Anspruch 1 oder von deren physiologisch unbedenklichen Salzen zur Herstellung eines Arzneimittels.

7. Verbindungen der Formel I nach Anspruch 1 oder deren physiologisch unbedenklichen Salze zur Verwendung bei der Bekämpfung von Krankheiten.

8. Verwendung von Verbindungen der Formel I nach Anspruch 1 zur Herstellung von Arzneimitteln zur Behandlung von Arrhythmien, Angina pectoris, Infarkten sowie zur präventiven Behandlung der genannten Indikationen.

## Claims

1. 4-Mercaptobenzoylguanidines of the formula I 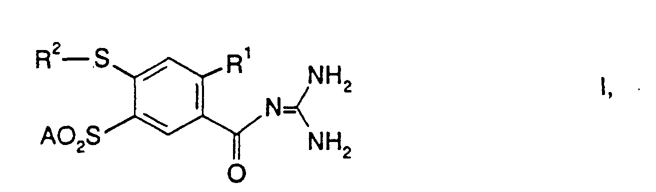 in which
R¹ is A, CF₃, CH₂F, CHF₂ or C₂F₅,
R² is H, A, cycloalkyl having 3 to 7 carbon atoms, Ph or Het,
Het is a mono- or dicyclic saturated, unsaturated or aromatic heterocycle having 1 to 4 nitrogen, oxygen and/or sulfur atoms, attached via N or C, which can be unsubstituted or mono-, di- or trisubstituted by Hal, CF₃, A, OH, OA, SH, SA, NH₂, NHA, NA₂, CN, NO₂ and/or carbonyl oxygen,
A is alkyl having from 1 to 6 carbon atoms,
Hal is F, Cl, Br or I, and
Ph is unsubstituted phenyl or phenyl which is mono-, di- or trisubstituted by A, OA, NH₂, NHA, NA₂, F, Cl, Br and/or CF₃,
and the physiologically unobjectionable salts thereof.

2. (a) Diaminomethylene-2-methyl-4-(4-pyridylthio)-5-methyl-sulfonylbenzamide;
(b) N-diaminomethylene-2-methyl-4-methylthio-5-methylsulfonylbenzamide;
(c) N-diaminomethylene-2-methyl-4-isopropylthio-5-methylsulfonyl-benzamide;
(d) N-diaminomethylene-2-methyl-4-phenylthio-5-methylsulfonylbenzamide;
(e) N-diaminomethylene-2-methyl-4-cyclohexylthio-5-methylsulfonylbenzamide;
(f) N-diaminomethylene-2-methyl-4-(3-chloro-phenylthio)-5-methylsulfonylbenzamide;
(g) N-diaminomethylene-2-ethyl-4-(4,5-dihydrothiazol-2-yl-thio)-5-methylsulfonylbenzamide
according to Claim 1, and physiologically unobjectionable salts thereof.

3. Process for the preparation of alkylbenzoyl-guanidine derivatives of the formula I according to Claim 1 and of salts thereof, characterized in that a compound of the formula II 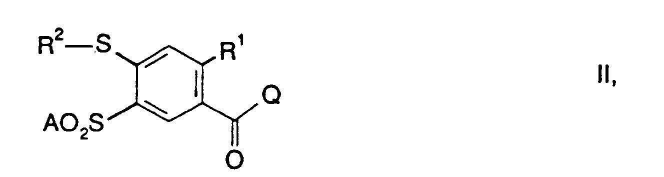 in which R¹, R² and A have the meanings given above
and
Q is Cl, Br, OA, O-CO-A, O-CO-Ph, OH or another reactive esterified OH group or a leaving group which can readily be substituted nucleophilically,
is reacted with guanidine,
or in that a benzoylguanidine of the formula III 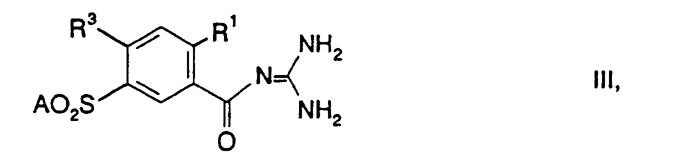 in which R¹ and A have the meanings given above
and
R³ is F, Cl, Br, I or another suitable leaving group,
is reacted with a compound of the formula IV
R²-S-H IV
in which
R² has the meanings given,
or with a salt-like compound which can be derived therefrom, or with a thiolate,
or in that a compound which otherwise corresponds to the formula I but which contains, instead of one or more hydrogen atoms, one or more reducible groups and/or one or more additional C-C and/or C-N bonds, is treated with a reducing agent,
or in that a compound which otherwise corresponds to the formula I but which contains, instead of one or more hydrogen atoms, one or more solvolysable groups is treated with a solvolysing agent
and/or in that a base of the formula I which is obtained is converted by treatment with an acid into one of its salts.

4. Process for the production of pharmaceutical preparations, characterized in that a compound of the formula I according to Claim 1 and/or one of its physiologically unobjectionable salts is brought, together with at least one solid, liquid or semiliquid excipient or auxiliary, into a suitable dosage form.

5. Pharmaceutical preparation, characterized by a content of at least one compound of the general formula I according to Claim 1 and/or one of its physiologically unobjectionable salts.

6. Use of compounds of the formula I according to Claim 1 or of the physiologically unobjectionable salts thereof for the preparation of a medicament.

7. Compounds of the formula I according to Claim 1 or the physiologically unobjectionable salts thereof for use in the control of diseases.

8. Use of compounds of the formula I according to Claim 1 for the preparation of medicaments for the treatment of arrhythmias, angina pectoris, infarctions and for the preventive treatment of the said indications.

## Revendications

1. 4-mercaptobenzoylguanidines de formule I 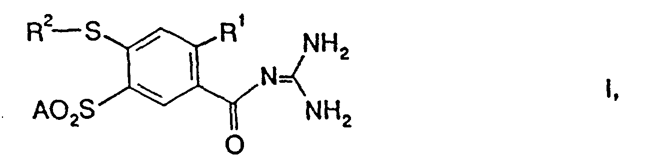 dans laquelle
R¹ représente A, CF₃, CH₂F, CHF₂ ou C₂F₅,
R² représente H, A, un groupe cycloalkyle ayant de 3 à 7 atomes de carbone, Ph ou Het,
Het représente un hétérocycle mono- ou bicyclique, saturé, insaturé ou aromatique, ayant de 1 à 4 atomes de N, O et/ou S, relié par N ou C, qui peut être non substitué ou une, deux ou trois fois substitué par Hal, CF₃, A, OH, OA, SH, SA, NH₂, NHA, NA₂, CN, NO₂ et/ou un atome d'oxygène en fonction carbonyle,
A représente un groupe alkyle ayant de 1 à 6 atomes de carbone,
Hal représente F, Cl, Br ou I, et
Ph représente un radical phényle non substitué ou substitué une, deux ou trois fois par A, OA, NH₂, NHA, Na₂, F, Cl, Br et/ou CF₃,
ainsi que leurs sels physiologiquement acceptables.

2. (a) diaminométhylène-2-méthyl-4-(4-pyridylthio)-5-méthylsulfonylbenzamide,
(b) N-diaminométhylène-2-méthyl-4-méthylthio-5-méthylsulfonylbenzamide,
(c) N-diaminométhylène-2-méthyl-4-isopropylthio-5-méthylsulfonylbenzamide,
(d) N-diaminométhylène-2-méthyl-4-phénylthio-5-méthylsulfonylbenzamide,
(e) N-diaminométhylène-2-méthyl-4-cyclohexylthio-5-méthylsulfonylbenzamide,
(f) N-diaminométhylène-2-méthyl-4-(3-chlorophénylthio)-5-méthylsulfonylbenzamide,
(g) N-diaminométhylène-2-éthyl-4-(4,5-dihydro-thiazol-2-yl-thio)-5-méthylsulfonylbenzamide
selon la revendication 1, ainsi que leurs sels physiologiquement acceptables.

3. Procédé pour la préparation de dérivés de type alkylbenzoylguanidine de formule 1 selon la revendication 1 ainsi que de leurs sels, caractérisé en ce que l'on fait réagir avec de la guanidine un composé de formule II 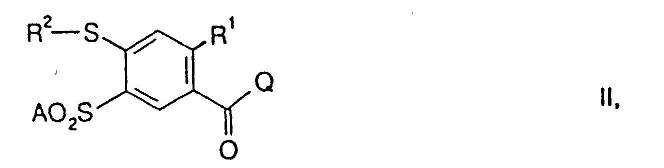 dans laquelle R¹, R² et A ont les significations indiquées précédemment, et
Q représente Cl, Br, OA, O-CO-A, O-CO-Ph, OH ou un autre groupe OH estérifié réactif, ou un groupe partant aisément remplaçable par substitution nucléophile,
ou en ce que l'on convertit en un thiolate une benzoylguanidine de formule III 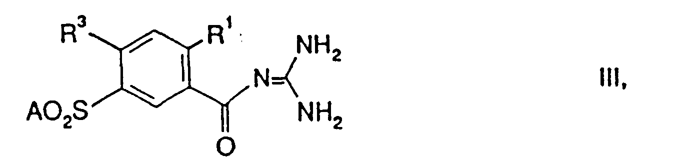 dans laquelle R¹ et A ont les significations indiquées précédemment
et
R³ représente F, Cl, Br, I ou un autre groupe partant approprié,
à l'aide d'un composé de formule IV
R²-S-H IV
dans laquelle R² a la signification indiquée,
ou d'un composé de type sel pouvant être obtenu à partir de celui-ci,
ou en ce que l'on traite par un réducteur un composé correspondant par ailleurs à la formule I mais qui, au lieu d'un ou plusieurs atomes d'hydrogène, comporte un ou plusieurs groupe(s) réductible(s) et/ou une ou plusieurs liaison(s) C-C ou C-N supplémentaire(s),
ou en ce que l'on traite par un agent de solvolyse un composé, correspondant par ailleurs à la formule I, mais qui, au lieu d'un ou plusieurs atomes d'hydrogène, comporte un ou plusieurs groupe(s) apte(s) à la solvolyse
et/ou en ce que l'on convertit en un de ses sels une base obtenue de formule I, par traitement par un acide.

4. Procédé pour la préparation de compositions pharmaceutiques, caractérisé en ce que l'on met sous une forme galénique appropriée un composé de formule I selon la revendication 1 et/ou un de ses sels physiologiquement acceptables, conjointement avec un véhicule ou adjuvant solide, liquide ou semi-liquide.

5. Composition pharmaceutique, caractérisée par une teneur en au moins un composé de formule générale I selon la revendication 1 et/ou un de ses sels physiologiquement acceptables.

6. Utilisation des composés de formule I selon la revendication 1 ou de leurs sels physiologiquement acceptables, pour la fabrication d'un médicament.

7. Utilisation des composés de formule I selon la revendication 1 ou de leurs sels physiologiquement acceptables, pour l'utilisation dans la lutte contre des maladies.

8. Utilisation de composés de formule I selon la revendication 1, pour la fabrication de médicaments destinés au traitement d'arythmies, de l'angine de poitrine, d'infarctus, ainsi qu'au traitement préventif des indications citées.
